# EUROPEAN PATENT SPECIFICATION

(11) **EP 1 765 802 B1**
(45) Date of publication and mention of the grant of the patent: **15.10.2008**
(21) Application number: 05768158.7
(22) Date of filing: 01.07.2005
(51) Int. Cl.: C07D 305/14

(54) **PROCESS FOR THE ISOLATION OF PACLITAXEL**
VERFAHREN ZUR ISOLIERUNG VON PACLITAXEL
PROCEDE SERVANT A ISOLER PACLITAXEL

(30) Priority: 02.07.2004 US 585401 P
(43) Date of publication of application: 28.03.2007
(73) Proprietor: IVAX Pharmaceuticals s.r.o., 747 70 Opava 9 (CZ)
(72) Inventor: BUCHTA, Martin, 74714 Ludgerovice (CZ); CVAK, Ladislav, 746 01 Opava-Zlatniky (CZ); SOBOTIK, Roman, 747 07 Opava 7 (CZ); STVERKA, Pavel, Velka Polom 747 64 (CZ)
(74) Representative: Gallagher, Kirk James
(86) International application number: PCT/US2005/023543
(87) International publication number: WO 2006/014356

(56) References cited:
- US-A1- 2002 151 579
- US-B1- 6 333 419
- YANG X ET AL: "Purification of taxol by industrial preparative liquid chromatography" JOURNAL OF CHROMATOGRAPHY A, ELSEVIER, AMSTERDAM, NL, vol. 813, no. 1, 10 July 1998 (1998-07-10), pages 201-204, XP004127080 ISSN: 0021-9673 cited in the application

## Description

### FIELD OF THE INVENTION

The present invention relates to a process for the purification by chromatography of paclitaxel from mixtures containing paclitaxel.

### BACKGROUND

Paclitaxel, formerly known as "taxol", is an important chemotherapeutic agent useful for the treatment of human ovarian, breast and lung tumors. It has shown promise for a number of human cancers and its clinical uses have been reported in several review articles, such as Rowinsky, E. K., Ann. Rev. Med. 48:353 1997; Van Hoff, D. D., Semin. Oncol. 24:3 (1997); DeFuria, M. D., Phytomedicine 4:273 (1997); and Eisenhauer, E. A., Vermorken, J. B., Drugs 55:5 (1998).

Paclitaxel is a natural compound and was first isolated by Wani, et al., from the bark of Pacific yew (Taxus brevifolia). J. Am. Chem. Soc. 93:2325 (1971) Since that time, researchers have recognized that paclitaxel exists in all other species of the *Taxus genus*, including European yew (*Taxus baccata*), Himalayan yew (*Taxus Wallichiana*), Chinese yew (*Taxus celebita*), Japanese yew (*Taxus cuspidata*), Canadian yew (*Taxus canadensis*), Mexican yew (*Taxus globosa*), Florida yew (*Taxus floridana*) and ornamental yew (*Taxus media*) and all of their hybrids and cultivars.

The structural formula of paclitaxel has been determined and is reproduced below.

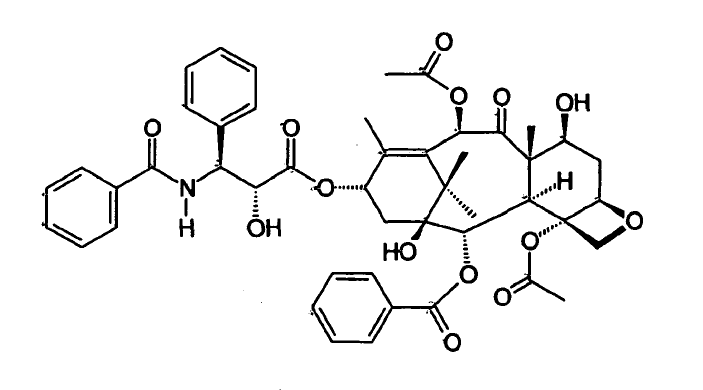

The isolation of paclitaxel from all types of potential vegetative sources is complex and difficult, partly due to its very low concentration in the biomass and partly due to the presence of other compounds, mainly taxanes, which possess similar properties to paclitaxel. Because of its high demand and low availability, additional potential sources for the industrial production of paclitaxel were highly sought (Cragg, G.M., et al., J. Nat. Prod. 56:1657 (1993)). Some of these sources include renewing parts of all types of plants from the *Taxus* genus (leaves, clippings, and hydroponically cultivated whole plants), fermenting *Taxus* tissue cultures, fermenting fungi parasitizing on *Taxus*, *etc*.

Typically, paclitaxel is isolated by first extracting a biomass with suitable solvents to obtain extracts that are raffinated by liquid-liquid extraction procedures. After the extraction procedures, paclitaxel isolation usually continues by a reversed phase chromatography step. Numerous examples of this technique can be found in the literature, including: Dauh-Rurng Wu, et al., J. Chrom. A, 702:233 (1995); Koppaka V. Rao, et al., Pharm. Res. 12:1003 (1995); and Xuefeng Yang, et al., J. Chrom. A, 813:201 (1998). Reverse phase chromatography is also described for the final purification of paclitaxel in several patents, such as: U.S. Patent No. 5,279,949; U.S. Patent No. 5,380,916; and U.S. Patent No. 5,969,165. A continuous process was also developed based on a simulated moving-bed reverse phase chromatography procedure. Dauh-Rurng Wu, et al., J. Chrom. A, 855:71 (1999). However, a disadvantage of using reverse phase chromatography is the need for water containing solvents, which can adversely cause the isomerization of paclitaxel to undesired 7-*epi*-paclitaxel.

The use of normal phase chromatography is also known for the isolation of paclitaxel, However, this process uses expensive stationary phases such as alkyl phenyl and pentafluor phenyl phases. Dauh-Rurng Wu, et al., J. Chrom. A. 702:233 (1995). More recent methods for isolating paclitaxel are based on complicated gradient elution procedures, or on several chromatographic steps using different mobile phase compositions, or on a combination of both normal phase and reversed phase chromatography. See, *e*.*g*., Young Kwang Park, et al,. J. Liq. Chrom. & Rel. Technol. 22(18):2755 (1999); Jun Xue, et al., J. Liq. Chrom. & Rel. Technol. 23(16):2499 (2000); U.S. Patent No. 5,478,736. Another approach is the use of normal phase chromatography using alumina as the adsorbent. Unfortunately, chlorinated organic solvents are often used as the mobile phase with alumina. Moreover, both epimerization and chemical decomposition of paclitaxel take place in this system, depending on the elution time. Zhiqiang Z., Zhiguo S., J. Liq. Chrom. & Rel. Technol. 23(17):2683 (2000).

Recently, a normal phase chromatography isolation of paclitaxel using a silica stationary phase and a carboxylic acid ester mobile phase was disclosed in U.S. Patent No. 6,333,419. The process described therein was designed for the separation of paclitaxel from cephalomannin.

There is, however, a continuing need for the large-scale isolation of paclitaxel from a variety of unwanted components with simple and efficient purification operations.

### SUMMARY OF THE DISCLOSURE

An advantage of the present invention is a simple, inexpensive and efficient method for the large scale isolation and production of high purity paclitaxel.

These and other advantages are satisfied, at least in part, by a process of isolating paclitaxel from paclitaxel mixtures using normal-phase chromatography based on a polyamide-based compound. The process includes applying a starting mixture comprising paclitaxel to a container comprising a polyamide-based compound and then applying a solution comprising one or more dialkyl ketones together with a less polar solvent to the container. The solution is added to cause it and the components of the mixture to elute from the container. One or more fractions of the eluting solution containing paclitaxel are then collected.

The process can advantageously be used for large-scale purification of paclitaxel. The starting paclitaxel material can be from any source that contains paclitaxel and some unwanted component. For example, the starting mixture can be a crude or purified extract obtained from vegetative sources, or by extracting cell cultures or bacteria strains. The starting mixture-can be a mixture of paclitaxel, cephalomannin and other taxanes, but it is not limited thereto.

Embodiments of the present invention include applying about one part by weight of the starting mixture to a container, *e*.*g*., a column, filled with more than about 20 parts by weight of a polyamide-based compound, *e*.*g*. polycaprolactam, polyundecanolactam, polylauryllactam, or poly(hexamethylene adipamide-*co*-caprolactam); applying a solution comprising acetone as the dialkyl ketone and either toluene or hexane as the less polar solvent; and increasing the concentration of the dialkyl ketone relative to the less polar solvent while applying the solution to the container. Pure paclitaxel or paclitaxel concentrate can then be isolated from the appropriate fractions containing the highest concentrations of paclitaxel by evaporation and crystallization.

Additional advantages of the present invention will become readily apparent to those skilled in this art from the following detailed description, wherein only the preferred embodiment of the invention is shown and described, simply by way of illustration of the best mode contemplated of carrying out the invention. As will be realized, the invention is capable of other and different embodiments, and its several details are capable of modifications in various obvious respects, all without departing from the invention. The present invention may be practiced without some or all of these specific details. In other instances, well known process operations have not been described in detail, in order not to unnecessarily obscure the present invention. Accordingly, the drawings and description are to be regarded as illustrative in nature, and not as restrictive.

### BRIEF DESCRIPTION OF THE DRAWINGS

Reference is made to the attached drawings, wherein:

Fig. 1 illustrates the composition of the starting material used in Example 1, as determined by HPLC analysis, wherein the abscissa represents the elution time in minutes, and the ordinate represents absorbance units.

Fig. 2 illustrates the composition of the main fractions of Example 1, as detemined by HPLC analysis, wherein the abscissa represents the elution time in minutes, and the ordinate represents absorbance units.

Fig. 3 illustrates the composition of the starting material used in Example 2, as detemined by HPLC analysis, wherein the abscissa represents the elution time in minutes, and the ordinate represents absorbance units.

Fig. 4 illustrates the composition of the main fractions of Example 2, which were eluted at about 40°C as detemined by HPLC analysis, wherein the abscissa represents the elution time in minutes, and the ordinate represents absorbance units.

Fig. 5 illustrates the composition of the main fractions of Example 2, which were eluted at about 70°C as detemined by HPLC analysis, wherein the abscissa represents the elution time in minutes, and the ordinate represents absorbance units.

Fig. 6 illustrates the composition of the starting material used in Example 3, as determined by HPLC, wherein the abscissa represents the elution time in minutes, and the ordinate represents absorbance units.

Fig. 7 illustrates the composition for the main fractions of Example 3, as detemined by HPLC, wherein the abscissa represents the elution time in minutes, and the ordinate represents absorbance units.

Fig. 8 illustrates the composition of the crystalline product of Example 3, as detemined by HPLC analysis, wherein the abscissa represents the elution time in minutes, and the ordinate represents absorbance units.

Fig. 9 illustrates the composition of the starting material used in Example 4, as determined by HPLC analysis, wherein the abscissa represents the elution time in minutes, and the ordinate represents absorbance units.

Fig. 10 illustrates the composition of the main fractions of Example 4, as detemined by HPLC analysis, wherein the abscissa represents the elution time in minutes, and the ordinate represents absorbance units.

Fig. 11 illustrates the composition of the starting material used in Example 5, as detemined by HPLC analysis, wherein the abscissa represents the elution time in minutes, and the ordinate represents absorbance units.

Fig. 12 illustrates the composition of the main fractions of Example 5, as detemined by HPLC analysis, wherein the abscissa represents the elution time in minutes, and the ordinate represents absorbance units.

Fig. 13 illustrates the composition of the starting material for Example 6, as detemined by HPLC analysis, wherein the abscissa represents the elution time in minutes, and the ordinate represents absorbance units.

Fig. 14 illustrates the composition of the main fractions of Example 6, as detemined by HPLC analysis, wherein the abscissa represents the elution time in minutes, and the ordinate represents absorbance units.

### DETAILED DESCRIPTION OF THE DISCLOSURE

The present invention was developed while investigating processes for the large-scale isolation of paclitaxel. After experimentation and investigation, it was-discovered that paclitaxel can be effectively purified by chromatography when the stationary phase comprises a polyamide-based adsorbent and when the mobile phase comprises a mixture of one or more dialkyl ketones together with another less polar co-solvent. The present invention advantageously allows the separation of paclitaxel from mixtures containing taxane impurities, which are otherwise difficult to remove but normally associated with paclitaxel mixtures.

As used in this specification, the singular forms "a," "an" and "the" specifically also encompass the plural forms of the terms to which they refer, unless the context clearly dictates otherwise.

The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%.

As used in this specification, whether in a transitional phrase or in the body of the claim, the terms "comprise(s)" and "comprising" are to be interpreted as having an open-ended meaning. That is, the terms are to be interpreted synonymously with the phrases "having at least" or "including at least". When used in the context of a process, the term "comprising" means that the process includes at least the recited steps, but may include additional steps. When used in the context of a compound or composition, the term "comprising" means that the compound or composition includes at least the recited features or components, but may also include additional features or components.

As used herein, the recitation of a numerical range for a variable is intended-to-convey that the invention may be practiced with the variable equal to any of the values within that range. Thus, for a variable that is inherently discrete, the variable can be-equal to any integer value of the numerical range, including the end-points of the range. Similarly, for a variable that is inherently continuous, the variable can be equal to any real value of the numerical range, including the end-points of the range. As an example, a variable which is described as having values between 0 and 2, can be 0, 1 or 2 for variables which are inherently discrete, and can be 0.0, 0.1, 0.01, 0.001, or any other real value for variables which are inherently continuous.

As used herein, unless specifically indicated otherwise, the word "or" is used in the "inclusive" sense of "and/or" and not the "exclusive" sense of "either/or."

Reference is made hereinafter in detail to specific embodiments of the invention. While the invention will be described in conjunction with these specific embodiments, it will be understood that it is not intended to limit the invention to such specific embodiments. In the following description, numerous specific details are set forth in order to provide a thorough understanding of the present invention. The present invention may be practiced without some or all of these specific details. In other instances, well known process operations have not been described in detail, in order not to unnecessarily obscure the present invention.

Any suitable materials and/or methods known to those of skill can be utilized in carrying out the present invention. However, preferred materials and methods are described. Materials, reagents and the like to which reference are made in the following description and examples are obtainable from commercial sources, unless otherwise noted

The starting paclitaxel mixture can be from any source that contains paclitaxel and some unwanted component. In practicing the present invention, any starting paclitaxel mixture can be used. For example, the starting mixture can be a mixture of paclitaxel, cephalomannin and other taxanes, but is not limited thereto. The starting mixture can also be a crude or partially purified extract of paclitaxel, which can be obtained from common sources such as by the extraction of fresh or dried bark, roots, leaves and/or branches or the whole plant of a paclitaxel containing plant, *e*.*g*. a *Taxus* plant. Other source of paclitaxel are also contemplated for use in practicing the present invention, including a crude or purified extract obtained from cell cultures of a cultivated *Taxus* plant, a fermentation broth prepared by cultivation of taxane-producing fungi; a fermentation broth prepared by cultivation of specific bacterial strains genetically modified for paclitaxel production.

The paclitaxel mixtures used in the invention can be of different origin and can contain a wide range of paclitaxel concentrations. Well defined mixtures having a high content of paclitaxel and other taxanes (cephalomannin, dihydrocephalomannin, taxol C, 7-*epi*-paclitaxel, *etc*.), are usually referred to as crude crystalline paclitaxel or crystalline paclitaxel concentrate, can be advantageously purified by an essentially single-step chromatography process employing a polyamide-based adsorbent. The starting material can be any paclitaxel concentrate prepared by known procedures from paclitaxel containing biomass. When using a complex starting material, the purification process may be complicated by the presence of non-taxane impurities. Starting with a complex paclitaxel mixture may result in the isolation of paclitaxel in a purity that is lower than desired. Hence, the present invention contemplates more than one purification step to purify paclitaxel such as a repeated chromatography step or a crystallization step.

In one aspect of the present invention, paclitaxel is separated from unwanted components by normal phase chromatography. In normal phase chromatography, a mixture to be purified is applied to a column or container holding an absorbent. A solvent or solvent mixture, *i*.*e*., a mobile phase, is subsequently applied to the column to cause the constituents of the mixture to pass through and elute from the column. The mobile phase can be gravity fed or applied by employing mechanical pumps and/or valves to maintain a more accurate control of the process, as is known in the art of chromatography. Under appropriate conditions, the constituents of the mixture are partitioned and eluted from the end of the column at different time intervals.

In practicing embodiments of the present invention, a paclitaxel mixture is applied to a container, *e*.*g*., a column, comprising a polyamide-based compound to initiate purification of the mixture. While it is believed that the polyamide-based compound employed in the present invention facilitates the separation of various constituents due to adsorption principles, it is not limited thereto or must necessarily act in accordance therewith. The principles by which paclitaxel is separated from a mixture in accordance with the present invention is not bound by any theory.

It is believed that the use of a polyamide-based adsorbent as a stationary phase in normal-phase chromatography is not common. However, it is believed that polyamides exhibit good chemical and physical stability in a wide range of organic solvents, which facilitates its use in separating components of a mixture. In accordance with embodiments of the present invention, the chromatographic process is based on a polyamide as the stationary phase. Although there are many potential variations in the chemical composition of a polyamide structure, the central structural unit - the amide bond (-CO-NH-)- is present in all cases. The order and number of methylene units between two amide bonds are believed responsible for the separation efficacy of a particular polyamide polymer.

Based on experimentation, it was determined that polylactams, and especially polycaprolactam, exhibit the best separation properties under the conditions employed. These materials showed a high degree of stability after repeated chromatography operations without a noticeable change in their separation efficacy. The stability and separation efficacy did not change even when a gradient elution step was used, *e*.*g*., when increasing the concentration of the dialkyl ketone relative to the less polar solvent during addition and elution of the solution to and from the column. The properties of a polyamide stationary phase contrasts with that of inorganic stationary phases, such as silica. For example, the switch from a polar to a non-polar mobile phase can be done very quickly without the need for either a large amount of a non-polar mobile phase for the elimination of the polar solvent from active centers or drying the stationary phase. The use of a polyamide in normal phase chromatography leads to a lower and constant swelling capacity as compared to its use in reversed phase chromatography, with a water containing mobile phase.

Polyamides are manufactured on an industrial scale in a wide range of particle sizes. The most suitable particle size for industrial scale separation is about 0.05-0.16 mm, which allows high throughput of the mobile phase at a low-pressure drop. Good separation of paclitaxel was achieved even when only about 20 weight parts of polyamide for about one part of the starting material was used. Nevertheless the quantity of the stationary phase depends on the composition of the starting material and desired purity of the final paclitaxel product. Suitable polyamides that can be used in practicing the present invention include polycaprolactam, polyundecanolactam, polylauryllactam, poly(hexamethylene adipamide-*co-*caprolactam), etc.

Another advantage of separating paclitaxel containing materials on a polyamide stationary phase is the improved separation selectivity observed with increasing temperature. It has been determined that employing higher temperatures during the process leads to an improvement in the separation between particular taxanes. It has been found that by maintaining the stationary or mobile phase at a temperature above ambient, *e*.*g*., at about 40-70°-C, the separation of paclitaxel from its analogue cephalomannin, which exhibits very similar separation behavior, is improved. Moreover, higher temperatures lower the viscosity of the mobile phase and, consequently, should lower production costs. Employing high temperature chromatography is not easily applicable to other separation processes, especially those using reversed phase chromatography because of the epimerization of paclitaxel in water containing solvents. In contrast, the epimerization of paclitaxel in a solution substantially excluding water or an alcohol is negligible and thus does not limit the process to lower temperatures.

The choice of suitable solvents for the mobile phase influences the separation efficacy. It was found that the best separation of paclitaxel from impurities present in a crude paclitaxel containing material was achieved with a gradient elution process. In one embodiment of the present invention, a paclitaxel mixture is applied to a polyamide compound followed by a first mobile phase, which comprises a low concentration of dialkyl ketone in the less polar solvent, *e*.*g*., where the solution comprises the dialkyl ketone in a ratio to the less polar solvent of about 5% (V/V) to about 15%(V/V). After a sufficient amount of the first mobile phase has been applied to the polyamide compound under isocratic conditions, *i*.*e*., without change to the solvent composition, a second mobile phase can be applied under a gradient condition, *i*.*e*., where the concentration of the dialkyl ketone is increased from a low concentration to a high concentration.

Under isocratic conditions all lipophilic substances are believed to elute from the column with the first mobile phase and when the concentration of the dialkyl ketone in the mobile phase is increased, it is believed that all remaining substances are eluted. This type of elution profile can effectively separate not only the non-taxane substances but also provides good separation for all substances possessing the taxane skeleton. In practicing certain embodiments of the present invention, paclitaxel has been observed to elute as the last substance from all common taxanes. Under these circumstances, it is believed that the displacement effect of paclitaxel occurs and it has been observed that the eluting zone of paclitaxel is sharp without overlap from any previous eluting substances. Consequently, the elution of paclitaxel as the last taxane can exclude "tag-along" effects, which have been a problem in the past.

Based on laboratory experiments, it was shown that paclitaxel can be effectively separated from other substances by chromatography employing a polyamide-based stationary phase and using mixtures of dialkyl ketones and a less polar organic co-solvent as the mobile phase. Suitable dialkyl ketones that can be used in practicing the present invention include acetone, methyl isobutyl ketone, 2-butanone, methyl ethyl ketone, etc. Acetone and methyl isobutyl ketone are the most preferred solvents among the group of dialkyl ketones. They are non-toxic and inexpensive. However, they tend to be too polar to be used alone as a single component mobile phase. Therefore the polarity of the mobile phase is decreased by the addition of one or more less polar co-solvents such as an aliphatic hydrocarbon, an aromatic hydrocarbon, or a dialkyl ether.

In an embodiment of the present invention, the boiling points of the solvents used as the mobile phase should be lower than about 130°C. This is because the product is preferable recovered from the chromatographic fractions by evaporation. If the boiling points of the solvents are too low however, evaporation of the solvents raises environmental concerns. Hence, the preferred less polar solvents used in practicing embodiments of the present invention include (C₅ - C₈) aliphatic hydrocarbons, such as hexane or heptane; (C₆ - C₈) aromatic hydrocarbons, such as toluene; (C₁ - C₄) dialkyl ethers, such as dibutyl ether; diisobutyl ether or *tert*-butyl methyl ether; or a mixture thereof.

Many solution combinations can be created from suitable solvents. To improve the efficiency of the process, the individual solvents can be recovered after the separation process, as by distillation. It is preferable to use a combination of solvents that have a large enough difference in their boiling points so as to facilitate their separation by distillation. Based upon its boiling point, toluene is the preferred solvent among the group of less polar solvents.

Another consideration regarding the choice of solvents to use as the mobile phase depends on the material to be separated. Usually complex mixtures with a low content of paclitaxel also contain some very polar impurities to be removed. Those impurities must ultimately be washed from the column. Such paclitaxel mixtures seem to be more readily separated by the combination of acetone and toluene as a mobile phase.

The eluted fractions containing a high concentration of paclitaxel can then be further isolated by vacuum evaporation. The extent of drying will dictate whether the product results in a viscous residue or a solid mass, either of which can be crystallized from suitable solvents to obtain paclitaxel in crystalline form. The use of a completely organic solvent system together with rapid evaporation of these solvents advantageously reduces or completely-eliminates the undesirable isomerization of paclitaxel to 7-*epi*-paclitaxel. This is another advantage over known isolation methods based on reverse phase chromatography.

### EXAMPLES

The following examples are intended to further illustrate certain preferred embodiments of the invention and are not limiting in nature. Those skilled in the art will recognize, or be able to ascertain, using no more than routine experimentation, numerous equivalents to the specific substances and procedures described herein.

### Example 1

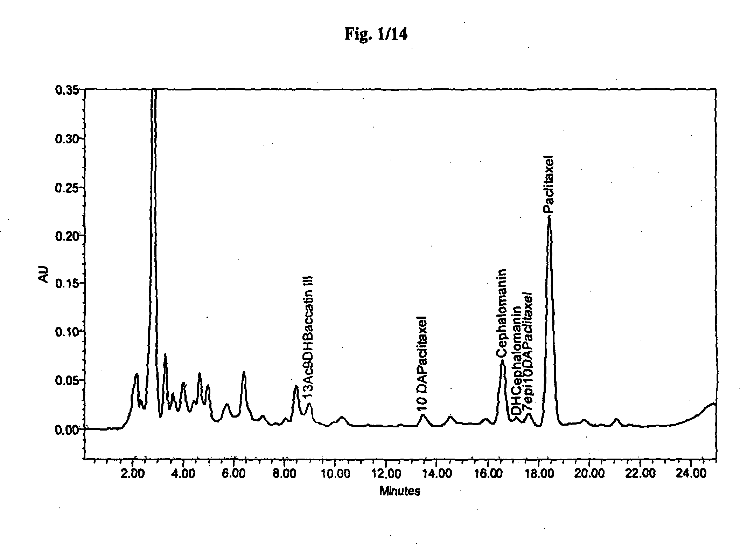

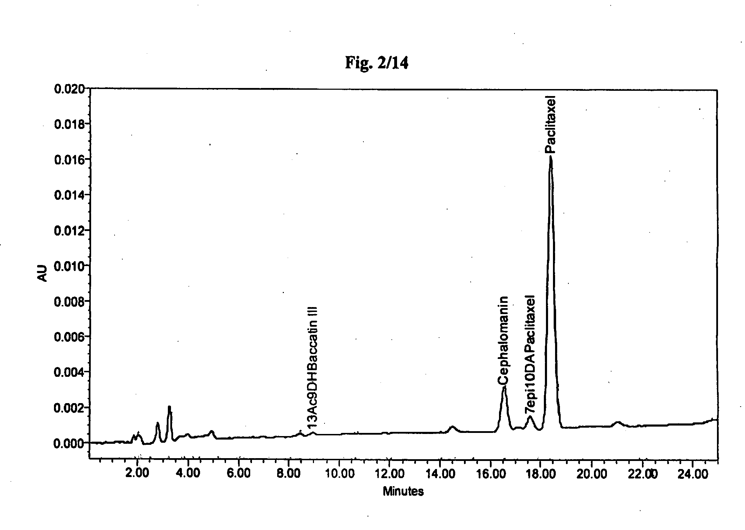

About 0.71 g of a crude mixture containing about 8.38 % of paclitaxel and about 2.23% of cephalomannin (as shown in Fig. 1) was dissolved in about 10 mL of a mixture containing acetone and toluene in a 10:90 (V/V) ratio. The solution was loaded on a column filled with about 45 g of a polyamide-based adsorbent (Polyamide Roth, particle size about 50 - 160 µm). The column was then eluted with about 600 mL of a 10:90 (V/V) acetone and toluene solution. Then about 1200 mL of a solution of acetone and toluene was added to the column while linearly increasing the gradient of acetone from a starting ratio of 10:90 (V/V) to final ratio of 100:0 (V/V). The flow of the mobile phase was maintained at about 50 mL/min during the entire elution procedure. Fractions of the eluting solution were taken approximatelyevery 50 mL from the time of injection to the end time of the gradient elution and analyzed by HPLC. Fractions of eluting solution containing paclitaxel were evaporated to dryness affording about 0.14 g of product containing about 37.54% of paclitaxel and about 6.80% of cephalomannin (HPLC analysis shown in Fig. 2).

### Example 2

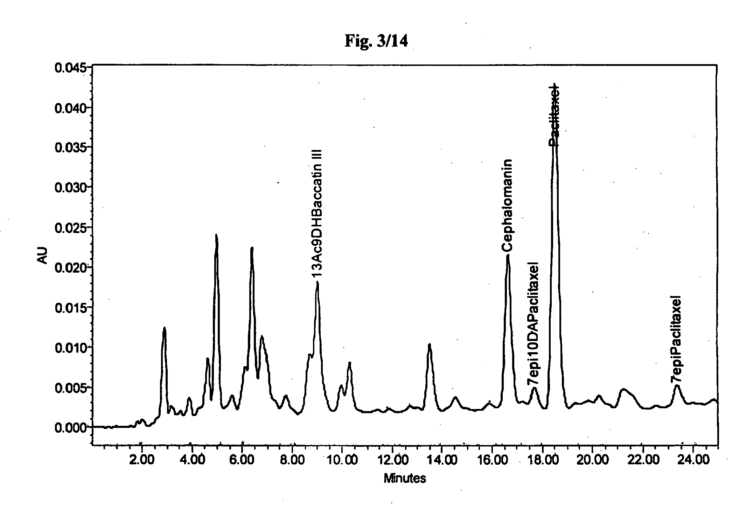

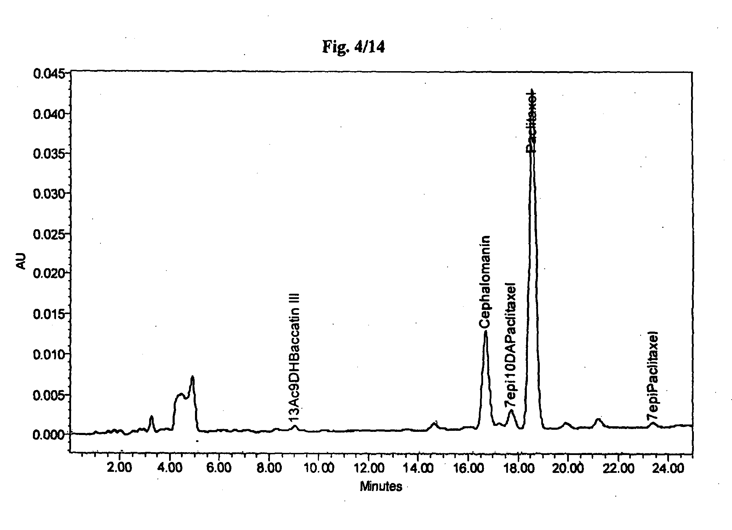

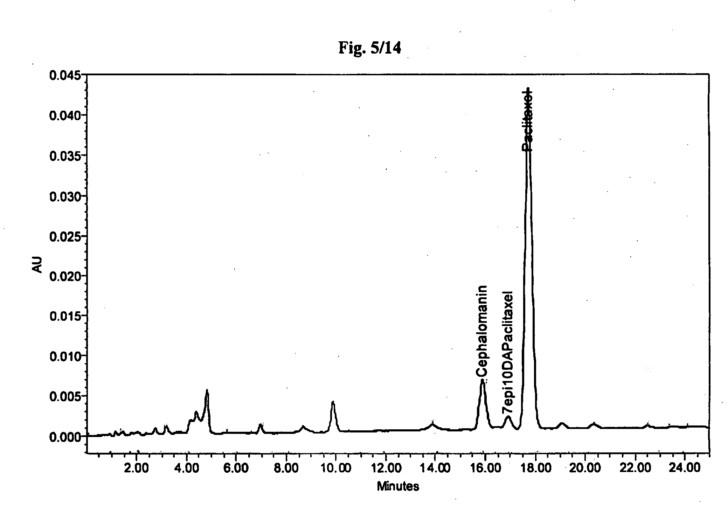

About 0.75 g of a crude mixture containing about 1.99% of paclitaxel and about 0.89% of cephalomannin (as shown in Fig. 3) was dissolved in 15 mL of a mixture of acetone and toluene 10:90 (V/V). The solution was loaded on a column filled with about 45 g of polyamide (Polyamide Roth, particle size about 50 - 160 µm). The column was then eluted with 600 mL of a 10:90 (V/V) acetone to toluene solution while maintaining a temperature of about 40°C. The approximately 1200 mL of an acetone and toluene combination was added while linearly increasing the gradient of acetone from a starting ratio of 10:90 (V/V) to final ratio of 100:0 (V/V). Fractions of eluting solution were taken at each 50 mL. The fractions were analyzed by HPLC. The same experiment with identical starting material was performed under the same conditions except the column was maintained at about 50°C, 60°C or 70°C. Corresponding fractions containing paclitaxel from all four separations were evaluated for both paclitaxel, and cephalomannin content. It was found that increasing temperature leads to separation improvement between these two substances from a ratio of about 3.89:1 at 40°C (HPLC analysis shown in Fig. 4) to a ratio of about 5.31:1 at 70°C (HPLC analysis shown in Fig. 5).

### Example 3

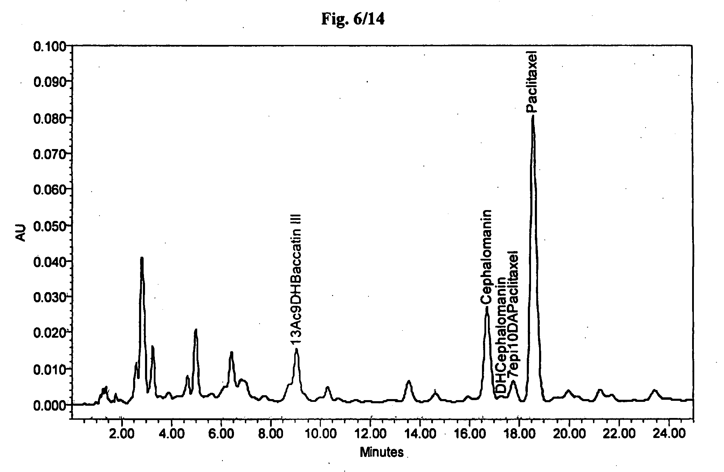

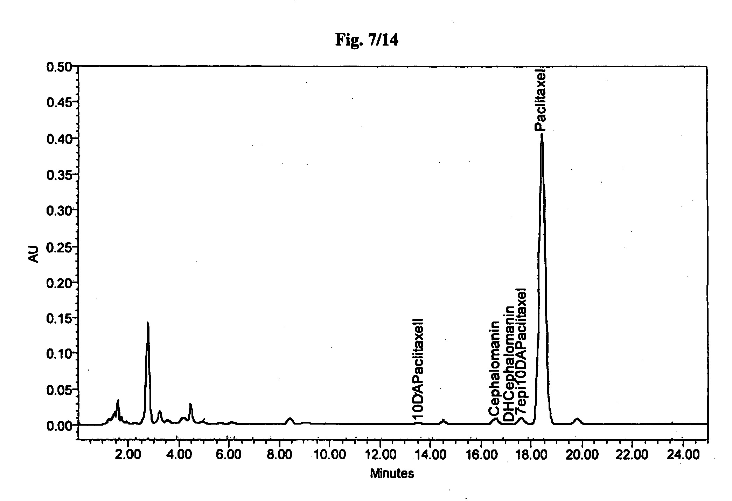

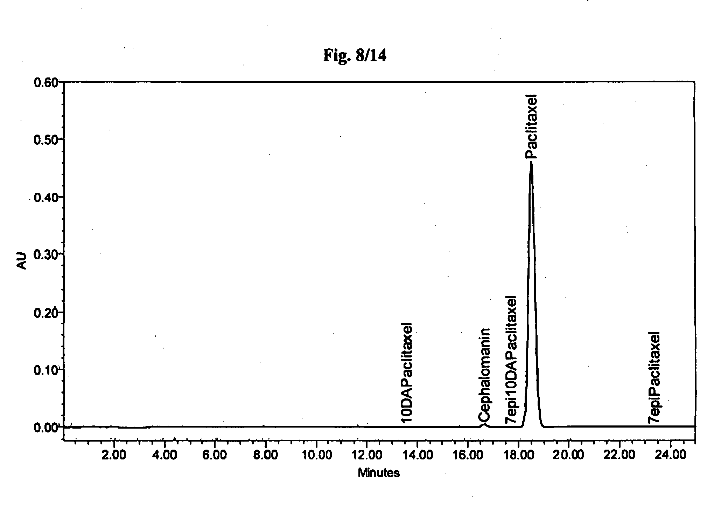

About 198.0 g of a crude paclitaxel mixture containing about 7.3% of paclitaxel and about 2.3% of cephalomannin (initial ratio paclitaxel/cephalomannin was about 3.17:1 according to HPLC analysis, as shown in Fig. 6) was dissolved in 4 L of acetone and toluene (10:90 V/V). The solution was loaded on a column filled with about 6830 g of polyamide (Polyamide Roth, particle size about 50 - 160 µm). The column was then eluted with 100 L of acetone and toluene (10:90 VN). It was further eluted with 200 L of mixture of acetone and toluene while linearly increasing the gradient of acetone from a starting ratio of 10:90 (V/V) to final ratio of 100:0 (V/V). Fractions of the eluting solution were taken each 20 L and analyzed by HPLC. Fractions containing pure paclitaxel were evaporated to dryness affording about 22.42 g of product containing about 60.5% of paclitaxel and about 1.3% of cephalomannin (HPLC analysis shown in Fig. 7). The purity of paclitaxel after crystallization from mixture of acetone and hexane (50:50 VN) was about 98.9% (HPLC analysis shown in Fig. 8).

### Example 4

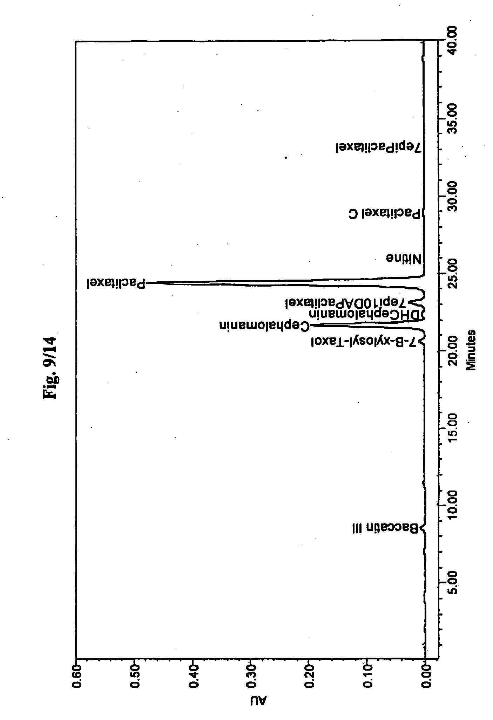

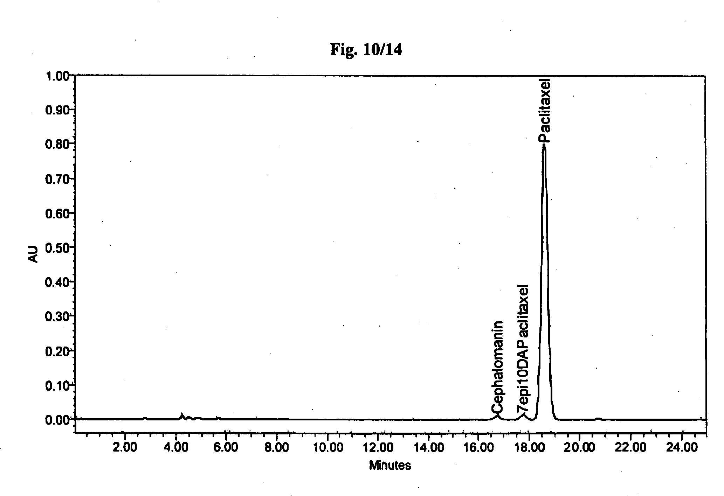

About 68.3 g of crude crystalline paclitaxel containing about 55.0 % of paclitaxel and about 19.0 % of cephalomannin (initial ratio paclitaxel/cephalomannin was approximately 2.89:1 according to HPLC analysis as shown in Fig. 9) was dissolved in 2 L of acetone and toluene (10:90 VN). The solution was then loaded on a column filled with about 6830.g of polyamide (Polyamide Roth, particle size 50 - 160 µm). The column was then eluted at 50°C with 100 L of acetone and toluene (10:90 VN) and further eluted with 200 L of acetone and toluene while linearly increasing the gradient of acetone from a starting ratio of 10:90 V/V) to a final ratio of 100:0 (VN). Fractions were taken each 20 L and analyzed by HPLC. Fractions containing pure paclitaxel were evaporated to dryness affording about 28.61 g of product containing about 83.1% of paclitaxel and about 1.0% of cephalomannin (HPLC analysis shown in Fig. 10).

### Example 5

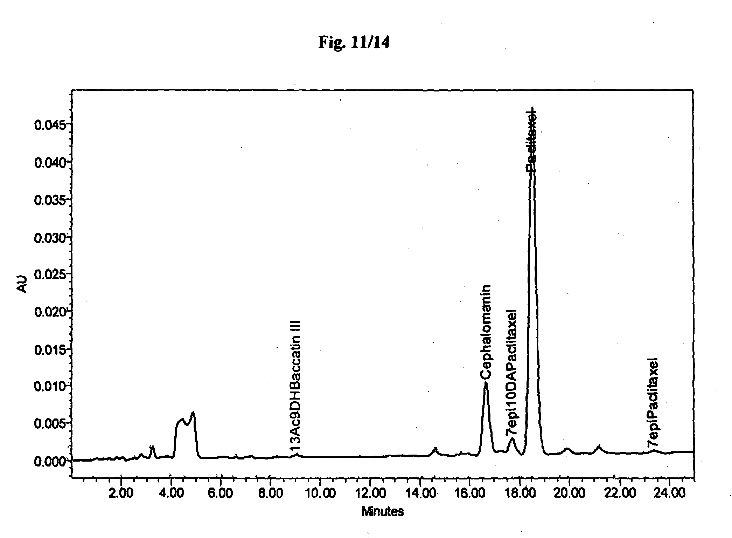

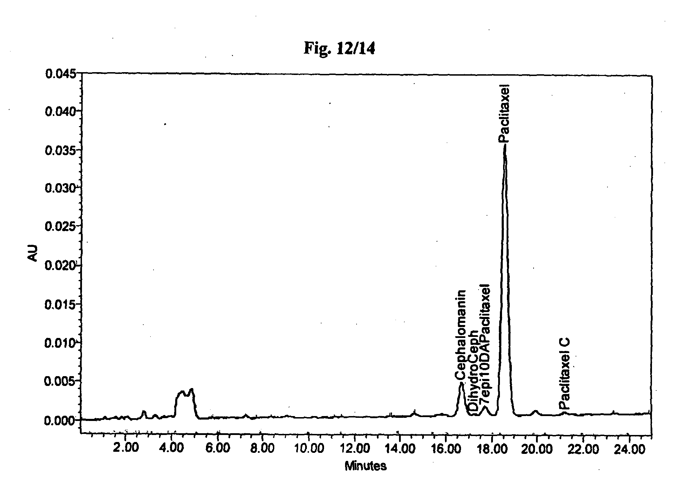

About 0.33 g of a crude paclitaxel mixture containing about 15.28% of paclitaxel and about 3.35% of cephalomannin (as shown in Fig. 11) was dissolved in 8 mL of acetone and hexane 31:69 (V/V). The solution was then loaded on a column filled with about 45 g of polyamide (Polyamide Roth, particle size about 50 - 160 µm). The column was then eluted with 600 mL of a mixture of acetone and hexane 20:80 (V/V). The column was then further eluted with 1200 mL of acetone and hexane while linearly increasing the gradient of acetone from a starting ratio of 20:80 (V/V) to a final ratio of 100:0 (V/V). Fractions of the eluting solution were taken each 50 mL and analyzed by HPLC. Fractions containing pure paclitaxel were evaporated to dryness affording about 0.07 g of product containing about 29.7% of paclitaxel and about 3.5% of cephalomannin (HPLC analysis shown in Fig. 12).

### Example 6

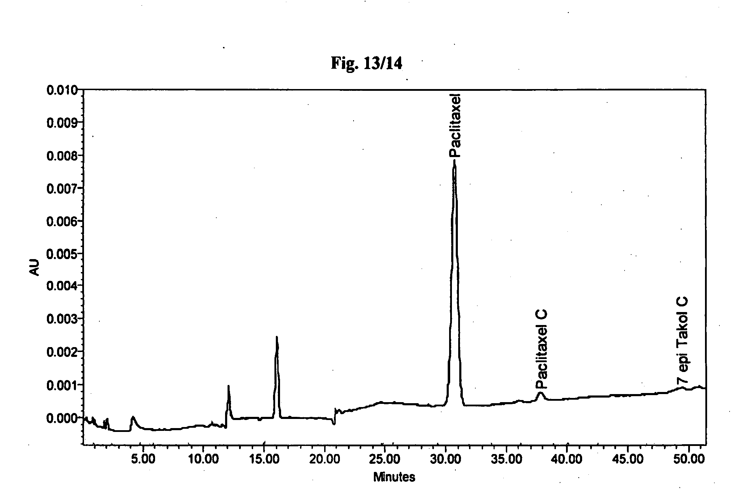

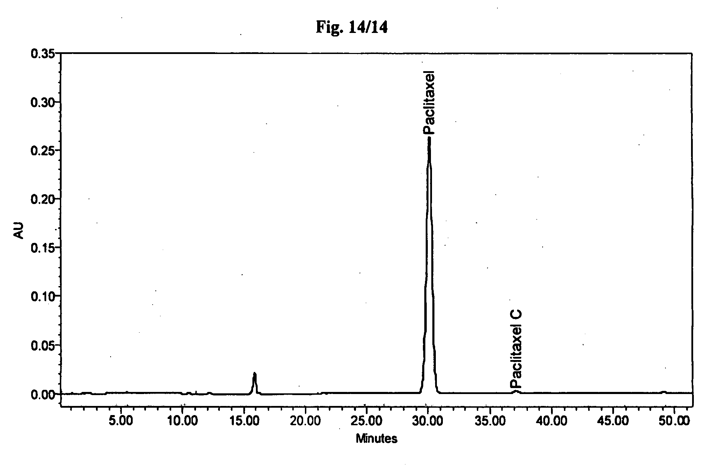

About 0.35 g of paclitaxel concentrate containing about 65.78% of paclitaxel and about 2.08% of taxol C (as shown in Fig. 13) was dissolved in 20 mL of acetone and toluene (15: 85 VN). The solution was then loaded on a column filled with about 35 g of polyamide (Polyamide Roth, particle size 315 µm). The column was then eluted with 750 mL of a mixture of acetone and toluene 15:85 (V/V). It was further eluted with 750 mL of a mixture of acetone and toluene while linearly increasing the acetone gradient from a starting ratio of 15:85 V/V) to final a ratio of 50:50 (VN). Fractions of the eluting solution were taken every 50 mL and analyzed by HPLC. Fractions containing pure paclitaxel were evaporated to dryness affording about 0.25 g of product containing about 68.63% of paclitaxel and about 0.63% of taxol C (HPLC analysis shown in Fig. 14).

In this disclosure there is described only the preferred embodiments of the invention and but a few examples of its versatility. It is to be understood that the invention is capable of use in various other combinations and environments and is capable of changes or modifications within the scope of the inventive concept as expressed herein. Thus, for example, those skilled in the art will recognize, or be able to ascertain, using no more than routine experimentation, numerous equivalents to the specific substances and procedures described herein. Such equivalents are considered to be within the scope of this invention, and are covered by the following claims.

## Claims

1. A process for the purification of paclitaxel, the process comprising the steps of:
a) applying a starting mixture comprising paclitaxel to a container comprising a polyamide-based compound;
b) applying to said container an eluting solution comprising one or more dialkyl ketones mixed with a less polar solvent to the container;
c) eluting the starting mixture; and
d) collecting one or more fractions of the eluting solution containing paclitaxel.

2. The process according to claim 1, wherein the starting mixture is a crude or purified extract obtained by extraction from the group consisting of a whole *Taxus* plant, fresh or dried bark, root, leaf or branch thereof.

3. The process according to claim 1, wherein the starting mixture is a crude or purified extract obtained by extracting cell cultures obtained from a *Taxus* plant.

4. The process according to claim 1, wherein the starting mixture is a crude or purified extract obtained by extraction of a fermentation broth prepared by cultivation of taxane-producing fungi.

5. The process according to claim 1, wherein the starting mixture is a crude or purified extract obtained by extraction of a fermentation broth prepared by cultivation of specific bacterial strains genetically modified for paclitaxel production.

6. The process according to claim 1, wherein the starting mixture is a mixture of paclitaxel, cephalomannin and other taxanes.

7. The process according to claim 1, wherein the one or more dialkyl ketones are chosen from the group consisting of acetone, 2-butanone, or methyl isobutyl ketone.

8. The process according to claim 1, wherein the less polar solvent is chosen from the group consisting of a (C₅ - C₈) aliphatic hydrocarbon, a (C₆ - C₈) aromatic hydrocarbon, and (C₁ - C₄) dialkyl ether or a mixture thereof.

9. The process according to claim 8, wherein the (C₅ - C₈) aliphatic hydrocarbon is hexane or heptane.

10. The process according to claim 8 wherein the (C₆ - C₈) aromatic hydrocarbon is toluene.

11. The process according to claim 8, wherein the (C₁ - C₄) dialkyl ether is chosen from the group consisting of dibutyl ether, diisobutyl ether and *tert*-butyl methyl-ether.

12. The process according to claim 1, wherein about one part by weight of the starting mixture is applied to a column filled with more than about 20 parts by weight of the polyamide-based compound.

13. The process according to claim 1, wherein the polyamide is chosen from the group consisting of polycaprolactam, polyundecanolactam and polylauryllactam.

14. The process according to claim 1, wherein the polyamide is poly(hexamethylene adipamide-co-caprolactam).

15. The process according to claim 1, wherein the solution comprises the one or more dialkyl ketones in a ratio to the less polar solvent of about 5% (V/V) to about 100% (V/V).

16. The process according to claim 15, wherein the solution comprises acetone as the one or more dialkyl ketones and either toluene or hexane as the less polar solvent.

17. The process according to claim 1 or claim 16, further comprising increasing the concentration of the dialkyl ketone to the less polar solvent while applying the solution to the container.

## Patentansprüche

1. Verfahren zum Aufreinigen von Paclitaxel, wobei das Verfahren die Stufen umfasst, bei denen man:
a) ein Ausgangsgemisch, welches Paclitaxel umfasst, in einen Behälter, der eine Verbindung auf Polyamidbasis umfasst, einbringt,
b) in den Behälter eine Elutionslösung, die ein oder mehrere Dialkylketone gemischt mit einem weniger polaren Lösungsmittel umfasst, in den Behälter einbringt,
c) das Ausgangsgemisch eluiert und
d) eine oder mehrere Fraktionen der Elutionslösung enthaltend Paclitaxel gewinnt.

2. Verfahren nach Anspruch 1, wobei das Ausgangsgemisch ein Rohextrakt oder ein aufgereinigter Extrakt ist, der erhalten wurde durch die Extraktion eines aus der Gruppe bestehend aus einer ganzen Taxus-Pflanze, frische/s/r oder getrocknete/s/r Rinde, Wurzel, Blatt oder Zweig davon.

3. Verfahren nach Anspruch 1, wobei das Ausgangsgemisch ein Rohextrakt oder ein aufgereinigter Extrakt ist, der erhalten wurde durch das Extrahieren von Zellkulturen, die aus einer *Taxus*-Pflanze erhalten wurden.

4. Verfahren nach Anspruch 1, wobei das Ausgangsgemisch ein Rohextrakt oder ein aufgereinigter Extrakt ist, der erhalten wurde durch die Extraktion einer Fermentationsbrühe, die hergestellt wurde durch die Kultivierung von Taxanproduzierenden Pilzen.

5. Verfahren nach Anspruch 1, wobei das Ausgangsgemisch ein Rohextrakt oder ein aufgereinigter Extrakt ist, der erhalten wurde durch die Extraktion einer Fermentationsbrühe, die erzeugt wurde durch die Kultivierung von spezifischen Bakterienstämmen, die für die Paclitaxel-Produktion genetisch verändert wurden.

6. Verfahren nach Anspruch 1, wobei das Ausgangsgemisch ein Gemisch von Paclitaxel, Cephalomannin und anderen Taxanen ist.

7. Verfahren nach Anspruch 1, wobei das eine oder die mehreren Dialkylketone ausgewählt sind aus der Gruppe, bestehend aus Azeton, 2-Butanon oder Methylisobutylketon.

8. Verfahren nach Anspruch 1, wobei das weniger polare Lösungsmittel ausgewählt ist aus der Gruppe, bestehend aus einem (C₅ - C₈)-aliphatischen Kohlenwasserstoff, einem (C₆ - C₈)-aromatischen Kohlenwasserstoff und einem (C₁ - C₄)-Dialkylether oder einem Gemisch davon.

9. Verfahren nach Anspruch 8, wobei der (C₅ - C₈)-aliphatische Kohlenwasserstoff Hexan oder Heptan ist.

10. Verfahren nach Anspruch 8, wobei der (C₆ - C₈)-aromatische Kohlenwasserstoff Toluol ist.

11. Verfahren nach Anspruch 8, wobei der (C₁ - C₄)-Dialkylether ausgewählt ist aus der Gruppe, bestehend aus Dibutylether, Diisobutylether und *tert-*Butylmethylether.

12. Verfahren nach Anspruch 1, wobei etwa ein Gewichtsteil des Ausgangsgemisches auf eine Säule, die mit mehr als etwa 20 Gewichtsteilen der Verbindung auf Polyamidbasis gefüllt ist, aufgebracht wird.

13. Verfahren nach Anspruch 1, wobei das Polyamid ausgewählt ist aus der Gruppe, bestehend aus Polycaprolactam, Polyundecanolactam und Polylauryllactam.

14. Verfahren nach Anspruch 1, wobei das Polyamid Poly(hexamethylenadipamid-co-Caprolactam) ist.

15. Verfahren nach Anspruch 1, wobei die Lösung das eine oder die mehreren Dialkylketone in einem Verhältnis zu dem weniger polaren Lösungsmittel von etwa 5% (V/V) bis etwa 100% (V/V) umfasst.

16. Verfahren nach Anspruch 15, wobei die Lösung Azeton als das eine oder die mehreren Dialkylketone und entweder Toluol oder Hexan als das weniger polare Lösungsmittel umfasst.

17. Verfahren nach Anspruch 1 oder Anspruch 16, bei dem man weiterhin die Konzentration des Dialkylketons zu dem weniger polaren Lösungsmittel erhöht, während die Lösung in den Behälter eingebracht wird.

## Revendications

1. Un procédé pour la purification de paclitaxel, le procédé comprenant les étapes consistant à :
a. appliquer un mélange de départ comprenant du paclitaxel à un conteneur comprenant un composé à base de polyamide ;
b. appliquer audit conteneur une solution d'élution comprenant une ou plusieurs cétones dialkyles mélangées avec un solvant moins polaire au conteneur ;
c. éluer le mélange de départ ; et
d. collecter une ou plusieurs fractions de la solution d'élution contenant le paclitaxel.

2. Le procédé selon la revendication 1, dans lequel le mélange de départ est un extrait brut ou purifié obtenu par extraction du groupe consistant en une plante entière *Taxus,* son écorce fraîche ou séchée, sa racine, sa feuille ou sa branche.

3. Le procédé selon la revendication 1, dans lequel le mélange de départ est un extrait brut ou purifié obtenu par extraction de cultures de cellules obtenue d'une plante *Taxus.*

4. Le procédé selon la revendication 1, dans lequel le mélange de départ est un extrait brut ou purifié obtenu par extraction d'une fermentation en milieu liquide par culture d'un champignon produisant du taxane.

5. Le procédé selon la revendication 1, dans lequel le mélange de départ est un extrait brut ou purifié obtenu par extraction d'une fermentation en milieu liquide préparée par culture de souches de bactérie spécifique modifiée génétiquement pour la production de paclitaxel.

6. Le procédé selon la revendication 1, dans lequel le mélange de départ est un mélange de paclitaxel, cephalomannine et autres taxanes.

7. Le procédé selon la revendication 1, dans lequel la ou les cétones dialkyles sont choisies dans le groupe consistant en l'acétone, le 2-butanone, ou 1a méthyl isobutyl cétone.

8. Le procédé selon 1a revendication 1, dans lequel le solvant moins polaire est choisi dans le groupe consistant en un hydrocarbure aliphatique en C₅ à C₈, une hydrocarbone aromatique en C₆ à C₈, et un éther dialkyle en C₁ à C₄ ou un mélange de ceux-ci.

9. Le procédé selon la revendication 8, dans lequel l'hydrocarbure aliphatique en C₅ à C₈ est l'hexane ou l'heptane.

10. Le procédé selon la revendication 8, dans lequel l'hydrocarbure aromatique en C₆ à C₈ est le toluène.

11. Le procédé selon la revendication 8, dans lequel l'éther dialkyle en C₁ à C₄ est choisi dans le groupe consistant en le dibutyl éther, le diisobutyl éther et le tert-butyl methyle éther.

12. Le procédé selon la revendication 1, dans lequel environ une partie en poids du mélange de départ est appliquée à une colonne remplie avec plus que environ 20 parties en poids du composé à base de polyamide.

13. Le procédé selon la revendication 1, dans lequel 1e polyamide est choisi dans le groupe consistant en le polycaprolactame, le polyundecanolactame et le polylauryllactame.

14. Le précédé selon la revendication 1, dans lequel dans lequel le polyamide est le poly(hexamethylène adipamide-co-caprolactame).

15. Le procédé selon la revendication 1, dans lequel la solution comprend la ou les cétones dialkyles dans un ratio au solvant moins polaire d'environ 5% (V/V) à environ 100% (V/V).

16. Le procédé selon la revendication 15, dans lequel la solution comprend de l'acétone en tant que la ou les cétones dialkyles et soit du toluène, soit de l'hexane en tant que solvant moins polaire.

17. Le procédé selon la revendication 1 ou la revendication 16, comprenant en outre l'accroissement de la concentration de la cétone dialkyle par rapport au solvant moins polaire pendant l'application de la solution au conteneur.
